# FASCICULE DE BREVET EUROPEEN

(11) **EP 0 794 746 B1**
(45) Date de publication et mention de la délivrance du brevet: **14.07.2004**
(21) Numéro de dépôt: 95941148.9
(22) Date de dépôt: 27.11.1995
(51) Int. Cl.: A61F 2/42, A61F 2/36, A61F 2/46, A61B 17/17

(54) **IMPLANT D'ARTICULATION ET ANCILLAIRE ADAPTE**
GELENKIMPLANTAT UND ZUGEHÖRIGE HILFSINSTRUMENTE
JOINT IMPLANT AND ANCILLARY THEREFOR

(30) Priorité: 28.11.1994 FR 9414475
(43) Date de publication de la demande: 17.09.1997
(73) Titulaire: LABORATOIRE DE PROTHESE DENTAIRE CHIBRAC S.A., 33000 Bordeaux (FR)
(72) Inventeur: BAUDET, Jacques, F-33400 Talence (FR); BAKHACH, Joseph, F-33000 Bordeaux (FR); CHIBRAC, Jean, F-33000 Bordeaux (FR); CASTAING, Cédric, F-33290 Blanquefort (FR); GUTFRIND, Bernard, F-33170 Gradignan (FR)
(74) Mandataire: Fantin, Laurent
(86) Numéro de dépôt international: PCT/FR1995/001563
(87) Numéro de publication internationale: WO 1996/016613

(56) Documents cités:
- EP-A- 0 337 901
- EP-A- 0 455 929
- WO-A-94/13214
- DE-A- 2 751 537
- DE-A- 3 917 285
- FR-A- 2 594 323
- FR-A- 2 676 641
- US-A- 4 997 445

## Description

La présente invention a pour objet un implant métacarpo-phalangien ou métatarso-phalangien ou interphalangiens ainsi que l'ancillaire nécessaire.

De nombreuses maladies telles que l'arthrose ou les rhumatismes et/ou des accidents conduisent à des dégradations de l'articulation métacarpo-phalangienne ou métatarso-phalangienne ou interphalangiennnes, plus particulièrement à la destruction des cartilages de ces articulations.

Cette dégradation peut conduire à un handicap sérieux par limitation de l'angle de flexion autorisé par l'articulation.

L'articulation métacarpo-phalangienne est du type condylien et possède deux degrés de liberté puisque le doigt peut se déplacer en flexion-extension, dans le plan sagittal et en inclinaison latérale, dans un plan frontal, lorsque les doigts sont en extension uniquement. En effet, lorsque le doigt est fléchi, les ligaments latéraux, par leur implantation excentrée, limitent et même interdisent les mouvements d'inclinaison latérale.

Les articulations interphalangiennes ne présentent qu'un seul degré de liberté dans le sens flexion-extension à l'exception de tout mouvement latéral.

En cas d'intervention, il convient que le résultat respecte ces différentes possibilités de mouvement mais également les amplitudes naturelles de ces mouvements.

On sait aussi que des tendons extenseurs disposés sur la face dorsale des doigts assurent les différents mouvements d'extension des doigts et l'accès par voie dorsale n'est pas souhaitable pour les interventions chirurgicales car ces tendons sont une gêne pour le praticien mais de plus le "décrochage" temporaire de ce tendon cause également des difficultés en ce sens qu'il oblige à une ouverture de dimensions beaucoup plus importantes et la réadaptation est plus longue car ce type de tendon est beaucoup plus sollicité.

On connaît dans la demande de brevet européen EP O 455 929 des implants concernant le même domaine médico-chirurgical qui comprennent deux éléments distincts prévus pour coopérer en appui l'un sur l'autre et implantés respectivement l'un à l'extrémité du métacarpien et l'autre à l'extrémité en vis à vis de la première phalange ou aux extrémités en vis à vis de deux phalanges contiguës.

On remarque que les deux éléments comprennent chacun un insert prévu pour pénétrer dans la matière spongieuse de l'os et une tête prévue pour former l'articulation proprement dite.

L'implantation est réalisée par intervention par voie dorsale, en fléchissant le doigt ou la phalange concernée pour permettre l'accès à l'articulation, ce qui est contraire à la méthode d'intervention préconisée.

De plus, on remarque que l'implantation rend nécessaire une résection importante de chacun des os de l'articulation concernée, résection réalisée à l'aide d'une scie oscillante, à la limite des points d'attache des ligaments latéraux. Cette résection s'avère incontournable compte tenu de l'épaisseur de chacun des éléments de l'implant, généralement supérieure à l'épaisseur des cartilages dégradés.

De plus les têtes sont en réalité des coiffes qui viennent par dessus la surface d'extrémité de l'os concerné, après résection, avec un insert de grandes dimensions. On comprend en effet que cet insert soit de grandes dimensions pour éviter que la coiffe tourne d'une part, ce qui affaiblit la résistance de l'os car l'alésage va au-delà du canal médullaire, et que cet insert soit de forme conique d'autre part, pour permettre un bon ancrage, ce qui complique très sérieusement l'usinage avant implantation. De plus lesdits inserts ont une forme aplatie pour assurer un effet d'anti-rotation, plus exactement une forme de lame-palette trapézoïdale, implantable par impaction dans un trou de forme sensiblement correspondante, à réaliser préalablement, ice qui est une opération délicate nécessitant systématiquement un scellement complémentaire.

L'inconvénient principal de cette méthode réside dans le fait que les os subissent une résection importante. Un autre inconvénient vient du fait que la itenue de chacun des éléments de l'implant est assurée par le seul blocage mécanique de l'insert, tant en rotation qu'en translation dans le sens de l'axe ilongitudinal de l'os sans que la tête participe à ce blocage. Seul l'insert assure l'immobilisation de chacun des éléments par rapport à l'os, il est donc le garant du bon fonctionnement de l'implant.

Dans la demande de brevet européen EP-A-455.929 l'épaisseur des éléments de la prothèse doit être très réduite, malgré la résection et surtout pour essayer de la limiter et le scellement doit être très efficace afin d'éviter toute mobilité des éléments de la prothèse par rapport aux os qui les portent. Aussi, il est prévu dans ce brevet européen précité, une épaisseur d'au maximum 1 mm et préférentiellement 0,5 mm. De même, la rugosité est réduite à des excroissances de 5 µm et mieux encore 2,5 µm.

Une telle prothèse est très délicate à réaliser et son positionnement par rapport aux os sur lesquels elle est implantée reste aléatoire.

On remarque également que cette demande ne prévoit aucune méthode d'implantation, ne résout pas le problème de l'accès dorsal ci-dessus évoqué et considéré comme préjudiciable à un bon fonctionnement ultérieur.

On connaît aussi par la demande de brevet allemand DE-A- 27 51 537, une prothèse de hanche qui présente une tête sphérique qui se loge dans un siège dit aussi condyle. Il s'agit d'une articulation dite condylienne au lieu d'une articulation trochléenne comme dans la présente invention.

Dans cette prothèse, il est prévu deux éléments, un premier qui est une coiffe venant se fixer sur la tête de fémur, en l'occurrence par vissage et un cotyle de profil conjugué de celui de la coiffe en sorte de coopérer et permettre une articulation. Ce cotyle est rapporté dans le logement naturel de l'os iliaque et simplement scellé car il est maintenu par l'appui de la tête de fémur.

Le but de l'invention est de proposer un implant de resurfaçage intégré, l'ancillaire nécessaire ainsi qu'une méthode d'implantation chirurgicale afin de montrer tout l'intérêt de la présente invention et surtout pour montrer la réelle possibilité d'implantation d'un tel implant. Cet implant, selon l'invention vise à remplacer uniquement la partie cartilagineuse de l'articulation avec un traumatisme aussi réduit que possible pour le patient tout en autorisant une consolidation et une récupération rapide avec des degrés de flexion angulaire proches des valeurs naturelles.

Ce nouvel implant respecte les structures anatomiques et il est le garant d'une bonne stabilité articulaire, ce qui facilite aussi la récupération et diminue le temps de rééducation.

De plus, la voie d'abord est latérale comme cela va être décrit, si bien que l'on mesure tout l'intérêt d'éviter une intervention sur le tendon extenseur, intervention qui peut être responsable par la suite d'adhérences nuisibles. Ce nouvel implant, l'ancillaire associé ainsi que la méthode mise en oeuvre permettent de préserver l'environnement ligamentaire.

A cet effet, selon l'invention, l'implant de resurfaçage évitant une résection osseuse, notamment pour une articulation métacarpo-phalangienne ou métatarso-phalangienne ou interphalangiennnes comprenant deux éléments proximal et distal, implantés respectivement à l'extrémité de chacun des os de l'articulation, composés chacun d'un ancrage et d'une tête d'articulation, se caractérise en ce que les têtes des éléments proximal et distal ont une épaisseur et un profil anatomique sensiblement identiques à ceux du cartilage en état, c'est à dire avant dégradation et sont prévues pour être encastrées dans un fraisage de l'os réalisé en fonction du profil desdites têtes, sensiblement en lieu et place desdits cartilages en sorte que les éléments de resurfaçage soient intégrés.

La tête de l'élément proximal a une section médiane longitudinale sensiblement en C, de façon à assurer un serrage élastique des faces de dessus et de dessous de l'os, la branche supérieure étant plus longue que la branche inférieure.

Il est aussi prévu des picots d'immobilisation, disposés de part et d'autre de l'ancrage de la tête de l'élément distal.

Selon une autre caractéristique, l'ancrage comprend des tiges crantées circonférentiellement et munies chacune d'au moins un trou transversal pour une fixation par ostéo-intégration.

Plus particulièrement, la face interne de chacune des têtes proximal et distal comporte un quadrillage d'accrochage des tissus.

Quant à la face externe de la tête distale, elle peut être revêtue d'un matériau très résistant à l'abrasion tel qu'une céramique.

Dans un mode de réalisation particulier, les éléments proximal et distal sont réalisés en alliage de titane/céramique.

L'invention a aussi pour objet l'ancillaire de mise en place de l'implant et cet ancillaire comprend, pour chacun des éléments proximal et distal, au moins l'un des éléments suivants :
- un guide de traçage,
- un guide d'essai de fraisage comprenant les têtes des éléments de l'implant sans ancrage,
- un guide de perçage, et/ou
- un guide d'essai de perçage comprenant les têtes des éléments de l'implant et des ancrages de plus petites dimensions non crantés.

Plus spécifiquement, le guide de traçage comprend un jonc au profil du fraisage à réaliser, solidaire d'un bâti prolongé par une tige de préhension, ce jonc ayant une génératrice en forme de lame, destinée à venir en contact avec l'os.

Le guide de perçage comprend une plaquette percée d'au moins un trou, adaptée à la forme du logement fraisé.

Selon un perfectionnement, les différents guides sont solidarisés à un outil de manipulation.

La présente invention est décrite ci-après en regard des dessins annexés qui représentent un mode préférentiel de réalisation non limitatif et sur lesquels les figures suivantes montrent :
- la figure 1, une vue en perspective d'un même os dont chacune des deux extrémités est équipée de l'élément correspondant de l'implant selon l'invention,
- les figures 2A et 2B, une vue de face des extrémités de l'os après fraisage et avant mise en place des éléments de l'implant,
- les figures 3A et 3B, des vues de face respectivement de l'élément proximal et distal de l'implant selon l'invention,
- les figures 4A et 4B, des vues en coupe médiane respectivement par un plan vertical et par un plan horizontal de l'élément proximal et de l'élément distal selon l'invention,
- les figures 5A et 5B, des vues de la face arrière respectivement des éléments proximal et distal,
- les figures 6A et 6B sont des vues en coupe par un plan médian de l'articulation après pose des éléments proximal et distal de l'implant, respectivement par un plan horizontal, c'est à dire parallèle au plan de la main et par un plan vertical, c'est à dire par un plan perpendiculaire à celui de la main,
- la figure 7 est une vue d'un doigt avec la voie d'abord et l'articulation luxée permettant d'expliquer la méthode d'intervention,
- les figures 8A et 8B sont des vues en perspective des guides de traçage, respectivement des éléments proximal et distal,
- la figure 9 montre une vue en perspective d'une partie du jonc de traçage du guide de traçage,
- la figure 10 montre une vue en perspective d'un outil de manipulation ergonomique des guides,
- la figure 11 montre la prise en main de l'outil de manipulation représenté sur la figure 10,
- les figures 12A et 12B montrent respectivement les guides d'essai des éléments proximal et distal de l'implant sans ancrage, après fraisage,
- les figures 13A et 13B montrent respectivement les guides de perçage après fraisage, pour les éléments proximal et distal, et
- les figures 14A et 14B montrent respectivement les guides d'essai des éléments proximal et distal de l'implant avec ancrage, après perçage.

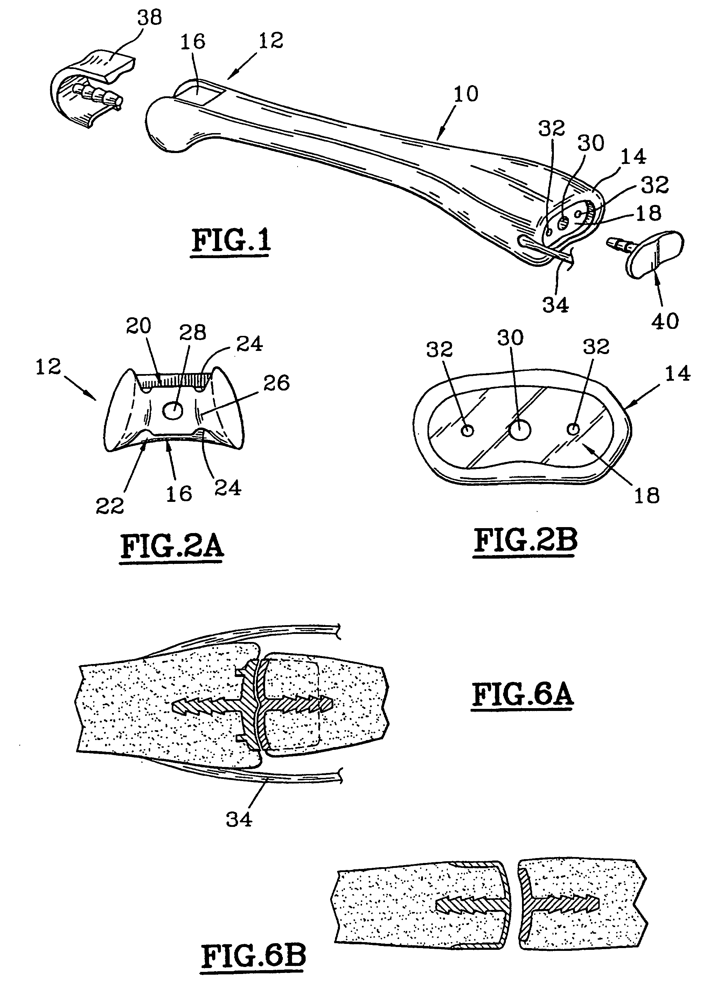

Sur la figure 1, on a représenté une phalange 10, dont les extrémités proximale 12 et distale 14 sont fraisées et portent respectivement un logement en creux 1 6 à section sensiblement en C et un logement en creux 18 prévus pour recevoir les éléments proximal et distal de l'implant selon l'invention. Pour des raisons de commodités, on a représenté les éléments proximal et distal sur une même phalange pour montrer leur positionnement respectif, mais il est bien entendu que les éléments sont en réalité portés chacun par les extrémités en vis à vis de deux phalanges articulées entre elles.

Le logement 16, a un profil sensiblement en C, les branches horizontales 20 et 22 de ce logement en C, c'est à dire les branches disposées dans le plan de la main, comprennent latéralement, comme montré sur la figure 2A, des rainures 24 qui se prolongent suivant l'axe longitudinal de la phalange. La branche 26 verticale de ce logement en C est munie d'un trou borgne 28, confondu sensiblement avec l'axe longitudinal de la phalange.

Le logement 18 est en creux et comporte à sa périphérie une partie osseuse résiduelle qui délimite ce logement en creux. Sensiblement au centre de ce logement, il est prévu un trou borgne 30, l'axe de perçage étant confondu avec l'axe longitudinal de la phalange.

Il est en outre ménagé deux trous borgnes 32, complémentaires, disposés de part et d'autre du trou central 30.

Les ligaments latéraux sont référencés 34.

Sur cette figure 1, on a également représenté les éléments proximal 38 et distal 40 de l'implant selon l'invention.

L'élément proximal 38 est représenté sur les figures 1, 3A, 4A, et 5A. Le profil de la tête 39 de cet élément proximal est sensiblement en C, avec une branche supérieure 42, correspondant à la face dorsale, plus longue que la branche inférieure 44, correspondant au côté palmaire et une branche verticale 46. Ce profil est conformé anatomiquement pour coopérer avec l'élément distal 40. La branche inférieure du côté palmaire est plus courte pour éviter le contact avec les ligaments.

Les branches dorsale et palmaire comportent également des bossages 48 et 50 prévus pour coopérer avec les rainures 24 du logement 16.

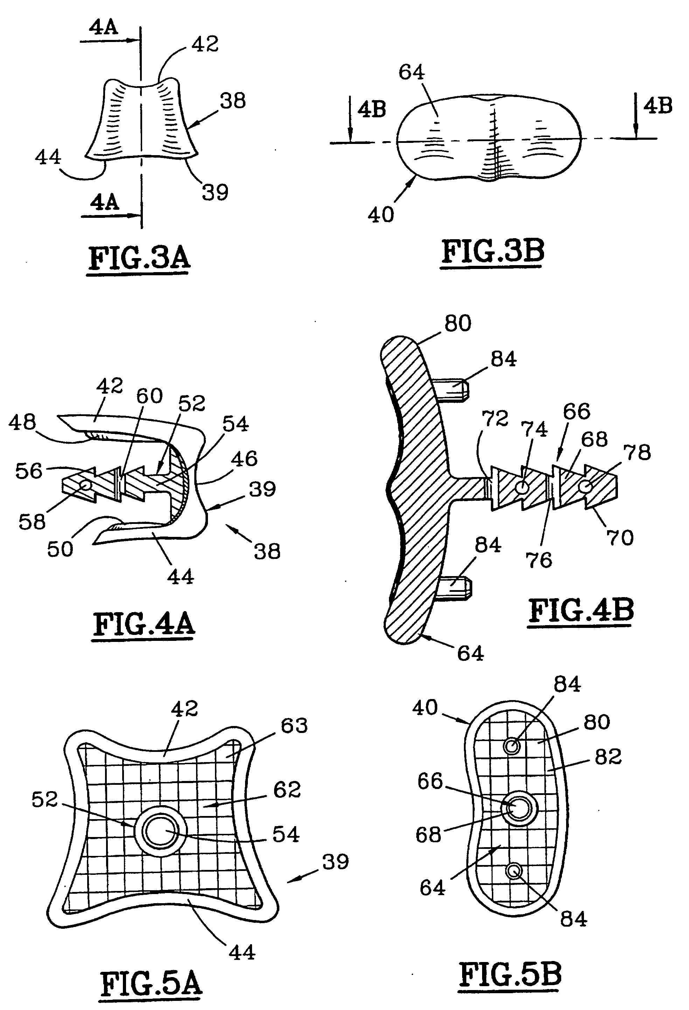

Ainsi que représenté sur la figure 4A, l'élément proximal 38 comprend un ancrage 52, plus particulièrement constitué d'une tige 54 avec des crans 56 circonférentiels et deux trous 58 et 60 orientés transversalement par rapport à l'axe longitudinal de la tige et orientés à 90° l'un par rapport à l'autre.

En se reportant à la figure 5A, on constate que la face interne 62 de la tête 39 porte un quadrillage 63 en relief, de façon à permettre une augmentation de la surface de contact et un meilleur recouvrement par les tissus après l'intervention.

L'élément distal 40, représenté sur les figures 3B, 4B et 5B, comprend une tête 64, légèrement concave et un ancrage 66, plus particulièrement une tige 68 avec des crans 70 circonférentiels dans laquelle sont ménagés quatre trous 72, 74, 76 et 78, orientés d'une part transversalement par rapport à l'axe longitudinal de la tige et d'autre part à 90°, alternativement, les uns par rapport aux autres.

La face interne 80 de la tête 64 porte également un quadrillage 82 en relief de façon à permettre une augmentation de la surface de contact et une meilleure couverture par les tissus de cette face interne en contact avec la matière osseuse, après intervention.

La face interne 80 comprend deux picots d'immobilisation 84, disposés de part et d'autre de la tige centrale 68, suivant un axe transversal.

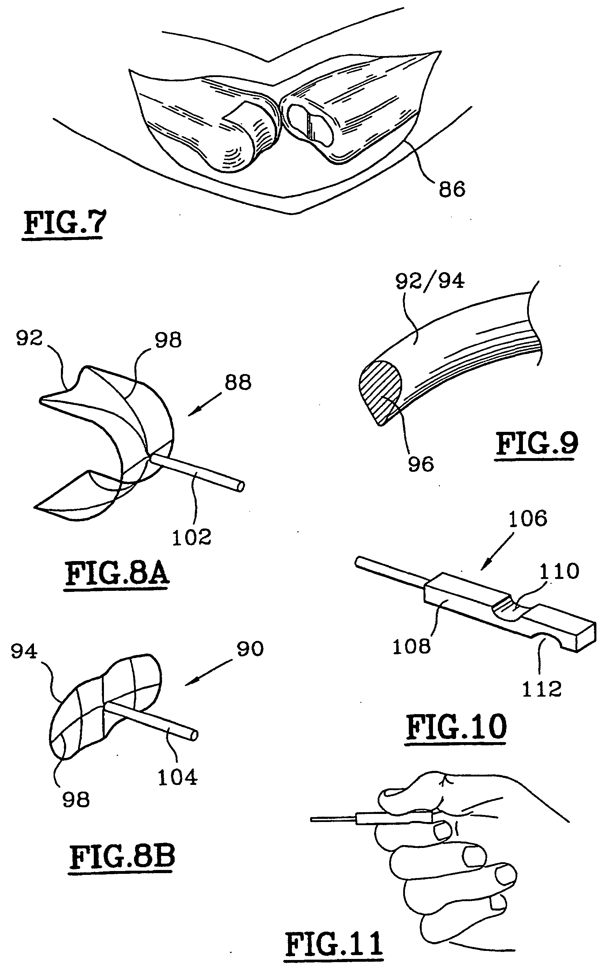

Sur les figures 6A et 6B, on a représenté une articulation équipée de l'implant selon l'invention suivant des plans, l'un parallèle au plan de la main et l'autre perpendiculaire.

Sur la figure 7, on a représenté la même articulation mais dans un contexte dans lequel c'est le doigt entier qui est montré avec la voie d'abord 86, permettant d'expliquer la méthode d'intervention chirurgicale pour la mise en place de l'implant selon l'invention.

En effet, la méthode d'implantation consiste à accéder latéralement à l'articulation, en incisant sur le côté du doigt, plus spécialement sur le côté orienté vers le pouce en sorte de pouvoir luxer la partie du doigt vers l'extérieur, après avoir détaché le ligament latéral rendu ainsi accessible par l'incision. Cet intervention est une opération courante qui laisse peu de traumatismes car ce ligament latéral est beaucoup moins sollicité que le tendon extenseur. Le choix de l'implant et l'étude des surfaces de l'os à travailler ont été effectués sur un cliché radio, de préférence grandeur nature.

Après la luxation, le praticien procède au travail des extrémités des phalanges à l'aide de l'ancillaire qui est décrit ci-après.

Tout d'abord, après détermination de la taille d'implant à mettre en place, le praticien procède dans une première étape au traçage des zones à fraiser. Pour cela il utilise pour chaque élément de l'implant, un guide de traçage 88 et 90, tel que représenté sur les figures 8A et 8B. Chacun de ces guide comprend un jonc périphérique 92, 94 de marquage dont la section est représentée agrandie sur la figure 9 pour montrer que la génératrice 96 du jonc destinée à venir au contact de l'os est conformée en lame.

Chaque jonc est solidaire d'un bâti filaire 98, 100 rigide, qui ne vient en aucun cas en contact avec l'os, ce bâti étant lui-même prolongé par une tige de préhension 102, 104.

Un perfectionnement de l'invention propose, ainsi que montré sur la figure 10 un outil de manipulation ergonomique 106, comprenant un corps 108 avec deux emplacements dessus 110 et dessous 112, décalés le long de l'axe longitudinal, pour disposer les doigts ainsi que montré sur la figure 11. Un tel outil est prévu pour être rapporté sur la tige de préhension 102 ou 104 des guides de traçage, ce qui facilite le travail du praticien ou de son assistant.

Par impaction ou par dépôt de bleu de méthylène dans lequel on trempe préalablement le jonc, le praticien marque l'os d'un tracé à suivre pour l'usinage.

Cette phase de traçage effectuée, le praticien fraise l'os sur une profondeur correspondant sensiblement à l'épaisseur de chacun des éléments de l'implant, dans l'espace délimité par le contour périphérique tracé. Il utilise pour cela du matériel connu couramment en service dans les services de chirurgie osseuse.

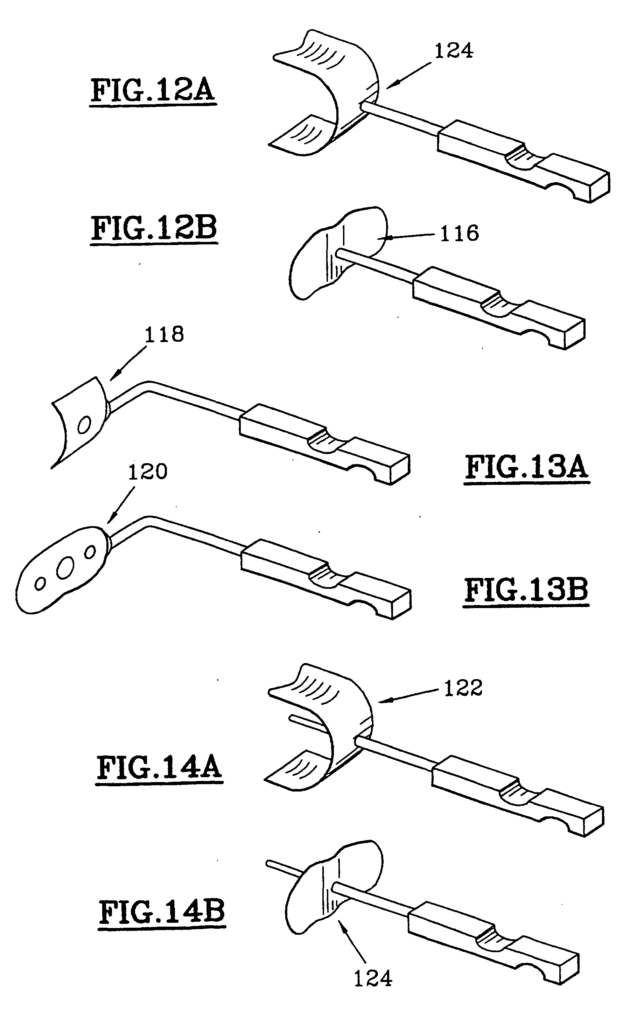

En plus de l'habileté propre à chaque praticien, il est prévu des guides d'essai 114, 116 pour contrôler au fur et à mesure l'avancement du fraisage. Ces guides d'essai sont représentés sur les figures 12A et 12B. Ils comprennent des éléments proximal et distal d'essai fixés sur un outil de manipulation 106, lesdits éléments proximal et distal étant prévus sans ancrage.

Après les différents essais et une fois les fraisages correctement réalisés, le praticien procède au perçage des trous destinés à recevoir les ancrages et à cet effet, il recourt aux guides de perçage 118 et 120 représentés sur les figures 13A et 13B. Ces guides comprennent chacun un gabarit conformé partiellement au profil de chacun des éléments proximal et distal de l'implant et muni d'au moins un trou formant canon de perçage.

Il reste au praticien à vérifier la bonne réalisation du perçage au moyen des guides d'essai complets 122, 124 comprenant des éléments proximal et distal avec ancrage mais ces ancrages sont lisses avec un diamètre légèrement inférieur au diamètre extérieur de l'ancrage définitif pour autoriser une pénétration aisée et un retrait facile sans dégradation de l'alésage réalisé. Pour des raisons pratiques, ces éléments sont montés également sur des outils de manipulation tels que décrit sur la figure 10.

Le praticien peut alors poser définitivement l'implant par introduction à friction douce de l'ancrage jusqu'à ce que les têtes viennent se positionner parfaitement dans leurs logements.

Le praticien ramène ensuite la partie de doigt luxé dans l'alignement naturel, il raccroche le ligament latéral par tout moyen convenable connu, puis il ferme la voie d'abord en recourant à des techniques connues en chirurgie et adaptées à ce type d'intervention.

Durant l'opération de fraisage, le praticien procède, si besoin est, à une retouche de la tête d'os dans le cas de présence d'excroissances, dues à la maladie, afin que le fonctionnement de l'articulation après mise en place de l'implant ne soit pas perturbé.

On remarque que les tendons fléchisseur et extenseur restent en position et ne sont pas concernés par l'intervention.

La face externe de l'élément distal peut être avantageusement recouverte d'une couche de matériau très résistant à l'abrasion, comme une céramique par exemple, qui facilite également le fonctionnement de l'articulation en diminuant le coefficient de frottement.

On peut aussi remarquer que les éléments proximal et distal sont immobilisés par leur propre forme une fois montés dans le fraisage correspondant. En effet, l'élément proximal est en C et les branches dorsale et palmaire constituent une pince. L'élément distal est encastré dans son logement, l'anti-rotation étant obtenue par le logement en creux et complétée par les picots d'immobilisation 84. De plus, les ligaments ne souffrent pas puisqu'ils ne subissent aucune distension au cours de l'intervention.

La description qui vient d'être faite l'a été pour les articulations métacarpo-phalangiennes et interphalangiennes mais elle s'applique sans modification à des opérations sur les articulations métatarso-phalangiennes et interphalangiennes du pied.

En cas de destruction plus profonde des éléments des surfaces articulaires, il est possible de recourir à un implant anatomique en faisant varier l'épaisseur de ces éléments pour rattraper la matière osseuse retirée ou dégradée.

Dans le mode de réalisation particulier qui vient d'être décrit, les éléments proximal et distal sont réalisés en alliage de titane/céramique mais ils peuvent être en chrome/cobalt, en titane carboné ou avec un revêtement en matériau de synthèse tel qu'un polymère.

L'invention s'applique également aux condyles, épaule, plus généralement à toute articulation de ce type.

## Revendications

1. Implant de resurfaçage évitant une résection osseuse, pour une articulation composée de deux extrémités d'os portant chacun un cartilage, comme une articulation métacarpo-phalangienne ou métatarso-phalangienne ou interphalangiennnes, ledit implant comprenant deux éléments, proximal (12) et distal (40), implantés respectivement à l'extrémité de chacun des os de cette articulation, chacun de ces éléments comprenant un ancrage (52,66) et une tête d'articulation (39,64), **caractérisé en ce que** les têtes des éléments proximal et distal ont une épaisseur et un profil anatomique sensiblement identiques à ceux du cartilage en état, c'est à dire celui du cartilage avant dégradation, la tête (39) de l'élément proximal (38) ayant une section longitudinale sensiblement en C sur toute sa largeur de la surface articulaire de l'implant, de façon à assurer un serrage élastique des faces de dessus et de dessous de l'os, la branche supérieure (42) étant plus longue que la branche inférieure (44), et **en ce que** les têtes sont prévues pour être encastrées chacune dans un fraisage de l'os réalisé en fonction du profil desdites têtes, en lieu et place desdits cartilages, en sorte que les éléments de resurfaçage soient intégrés.

2. Implant selon la revendication 1, **caractérisé en ce que** la tête (64) de l'élément distal (40) comprend des picots (84) d'immobilisation, disposés de part et d'autre de l'ancrage.

3. Implant selon l'une quelconque des revendications précédentes, **caractérisé en ce que** l'ancrage (52,66) comprend des tiges (54,68) crantées circonférentieliement (56,70) et munies chacune d'au moins un trou transversal pour une fixation par ostéo-intégration (58,60; 72,74,76 et 78).

4. Implant selon l'une quelconque des revendications précédentes, **caractérisé en ce que** la face interne (62,80) de chacune des têtes des éléments proximal et distal comporte un quadrillage (63,82) d'accrochage des tissus.

5. Implant selon l'une quelconque des revendications précédentes, **caractérisé en ce que** la face externe de la tête distale est revêtue d'un matériau très résistant à l'abrasion tel qu'une céramique.

6. Implant selon l'une quelconque des revendications précédentes, **caractérisé en ce que** les éléments proximal et distal sont réalisés en alliage de titane/céramique.

7. Ancillaire de mise en place de l'implant selon l'une quelconque des revendications 1 à 6, **caractérisé en ce qu'**il comprend, pour chacun des éléments proximal et distal, au moins l'un des éléments suivants :
- un guide de traçage (88,90) avec un jonc (92,94) au profil du fraisage à réaliser, solidaire d'un bâti (98) prolongé par une tige (102,104) de préhension,
- un guide d'essai (114,116) de fraisage comprenant les têtes des éléments de l'implant sans ancrage,
- un guide de perçage (118,120), et/ou
- un guide d'essai (122,124) de perçage comprenant les têtes des éléments de l'implant selon la revendication 1 et des ancrages de plus petites dimensions non crantés.

8. Ancillaire selon la revendication 9, **caractérisé en ce que** le jonc (92,94) comprend une génératrice (96) en forme de lame, destinée à venir en contact avec l'os.

9. Ancillaire selon la revendication 7 ou 8, **caractérisé en ce que** le guide de perçage (118,120) comprend un gabarit percé d'au moins un trou, adapté à la forme du logement fraisé.

10. Ancillaire selon l'une quelconque des revendications 7 à 9, **caractérisé en ce que** les différents guides sont solidarisés à un outil de manipulation (106).

## Patentansprüche

1. Implantat zur Oberflächenwiederherstellung, das eine Knochenresektion vermeidet, für ein Gelenk bestehend aus zwei Knochenenden, die jeweils einen Knorpel tragen, wie beispielsweise ein Metakarpophalangeal- oder Metatarsophalangeal- oder Interphalangealgelenk, wobei das Implantat zwei Elemente, ein proximales (12) und ein distales (40) umfasst, die jeweils am Ende jedes der Knochen dieses Gelenks implantiert werden, wobei jedes dieser Elemente eine Verankerung (52, 66) und einen Gelenkskopf (39, 64) umfasst, **dadurch gekennzeichnet, dass** die Köpfe der proximalen und distalen Elemente eine Dicke und ein anatomisches Profil aufweisen, die im Wesentlichen zu jenen des Knorpels im ursprünglichen Zustand, d.h. dem Zustand des Knorpels vor der Verschlechterung, identisch sind, wobei der Kopf (39) des proximalen Elements (38) einen im Wesentlichen C-förmigen Längsquerschnitt über seine gesamte Breite der Gelenksfläche des Implantats aufweist, um einen elastisches Einspannen der Ober- und Unterseiten des Knochens zu gewährleisten, wobei der obere Schenkel (42) länger als der untere Schenkel (44) ist, und dass die Köpfe dazu vorgesehen sind, jeweils in eine Fräsung des Knochens, die in Abhängigkeit vom Profil der Köpfe hergestellt ist, an Ort und Stelle der Knorpel eingesetzt zu werden, so dass die Elemente zur Oberflächenwiederherstellung integriert sind.

2. Implantat nach Anspruch 1, **dadurch gekennzeichnet, dass** der Kopf (64) des distalen Elements (40) Stippen (84) zur Festlegung umfasst, die beiderseits der Verankerung angeordnet sind.

3. Implantat nach einem der vorhergehenden Ansprüche, **dadurch gekennzeichnet, dass** die Verankerung (52, 66) am Umfang (56, 70) gezahnte Stangen (54, 68) umfasst, die jeweils mit mindestens einem Querloch für eine Befestigung durch Osteointegration (58, 60; 72, 74, 76 und 78) versehen sind.

4. Implantat nach einem der vorhergehenden Ansprüche, **dadurch gekennzeichnet, dass** die Innenseite (62, 80) jedes der Köpfe des proximalen und distalen Elements ein Gitternetz (63, 82) zur Befestigung der Gewebe umfasst.

5. Implantat nach einem der vorhergehenden Ansprüche, **dadurch gekennzeichnet, dass** die Außenseite des distalen Kopfes mit einem sehr abriebfesten Material, wie beispielsweise einer Keramik, überzogen ist.

6. Implantat nach einem der vorhergehenden Ansprüche, **dadurch gekennzeichnet, dass** das proximale und distale Element aus einer Legierung von Titan/Keramik hergestellt sind.

7. Zusatzgerät zum Einsetzen eines Implantats nach einem der Ansprüche 1 bis 6, **dadurch gekennzeichnet, dass** es für jedes proximale und distale Element mindestens eines der folgenden Elemente umfasst:
- eine Führung zum Anzeichnen (88, 90) mit einem Ansatz (92, 94) im Profil der durchzuführenden Fräsung, der mit einem Gestell (98) verbunden ist, das durch eine Stange (102, 104) zum Angreifen verlängert ist,
- eine Führung zum Testen der Fräsung (114, 116), die die Köpfe der Elemente des Implantats ohne Verankerung umfasst,
- eine Führung zum Bohren (118, 120), und/ oder
- eine Führung zum Testen der Bohrung (122, 124), die die Köpfe der Elemente des Implantats nach Anspruch 1 und nicht gezahnte Verankerungen mit kleineren Abmessungen umfasst.

8. Zusatzgerät nach Anspruch 7, **dadurch gekennzeichnet, dass** der Ansatz (92, 94) eine Mantellinie (96) in Form eines Blättchens umfasst, die dazu bestimmt ist, mit dem Knochen in Kontakt zu kommen.

9. Zusatzgerät nach Anspruch 7 oder 8, **dadurch gekennzeichnet, dass** die Führung zum Bohren (118, 120) eine mit mindestens einem Loch durchbohrte Schablone umfasst, die an die Form der gefrästen Aufnahme angepasst ist.

10. Zusatzgerät nach einem der Ansprüche 7 bis 9, **dadurch gekennzeichnet, dass** die verschiedenen Führungen mit einem Bedienungswerkzeug (106) verbunden sind.

## Claims

1. Resurfacing implant avoiding bone resection, for a joint made up of two bone ends, each of them with cartilage, such as a metacarpophalangeal or metatarsophalangeal or interphalangeal joint, said implant comprising two elements, namely a proximal element (12) and a distal element (40), which are implanted respectively at the end of each of the bones of this joint, each of these elements comprising an anchoring means (52, 66) and a joint head (39, 64), **characterized in that** the heads of the proximal and distal elements have a thickness and an anatomical profile substantially identical to those of the intact cartilage, that is to say of the cartilage before degradation, the head (39) of the proximal element (38) having a substantially C-shaped longitudinal section over the entire width of the articular surface of the implant, in such a way as to ensure elastic clamping of the top and bottom faces of the bone, the upper branch (42) being longer than the lower branch (44), and **in that** the heads are intended to be fitted in each case into a reamed recess in the bone, shaped to match the profile of said heads, in place of said cartilage, such that the resurfacing elements are integrated.

2. Implant according to Claim 1, **characterized in that** the head (64) of the distal element (40) comprises immobilizing studs (84) arranged on each side of the anchoring means.

3. Implant according to either of the preceding claims, **characterized in that** the anchoring means (52, 66) comprises rods (54, 68) notched circumferentially (56, 70) and each equipped with at least one transverse hole for fixation by osseointegration (58, 60; 72, 74, 76 and 78).

4. Implant according to any one of the preceding claims, **characterized in that** the inner face (62, 80) of each of the heads of the proximal and distal elements comprises a gridwork (63, 82) for attachment of the tissues.

5. Implant according to any one of the preceding claims, **characterized in that** the outer face of the distal head is coated with a material very resistant to abrasion, for example a ceramic.

6. Implant according to any one of the preceding claims, **characterized in that** the proximal and distal elements are made of an alloy of titanium/ceramic.

7. Ancillary for fitting the implant according to any one of Claims 1 to 6, **characterized in that** it comprises, for each of the proximal and distal elements, at least one of the following elements:
- a tracing guide (88, 90) with a rim (92, 94) having the profile of the reamed recess to be formed, and integral with a framework (98) continued by a grip rod (102, 104),
- a reaming test guide (114, 116) comprising the heads of the elements of the implant without anchoring means,
- a drilling guide (118, 120) and/or
- a drilling test guide (122, 124) comprising the heads of the elements of the implant according to Claim 1 and anchoring means of smaller dimensions and unnotched.

8. Ancillary according to Claim 7, **characterized in that** the rim (92, 94) comprises a generatrix (96) of blade shape intended to come into contact with the bone.

9. Ancillary according to Claim 7 or 8, **characterized in that** the drilling guide (118, 120) comprises a jig drilled with at least one hole, adapted to the shape of the reamed recess.

10. Ancillary according to any one of Claims 7 to 9, **characterized in that** the different guides are connected to a manoeuvring tool (106).
